# EUROPÄISCHE PATENTANMELDUNG

(11) **EP 0 716 069 A1**
(43) Veröffentlichungstag der Anmeldung: **12.06.1996**
(21) Anmeldenummer: 95118696.4
(22) Anmeldetag: 28.11.1995
(51) Int. Cl.: C07C 43/225, C07C 41/22

(54) **Verfahren zur Herstellung von Alkyl-4-bromphenylethern**

(30) Priorität: 07.12.1994 DE 4443592
(71) Anmelder: Great Lakes Chemcial Konstanz GmbH, D-78467 Konstanz (DE)
(72) Erfinder: Lehman, Bernd, Dr., D-78465 Konstanz (DE)
(74) Vertreter: Wibbelmann, Jobst, Dr., Dipl.-Chem.

(57) **Zusammenfassung**

Die Erfindung betrifft ein Verfahren zur Herstellung von Alkyl-4-bromphenylethern bei dem ein Alkylphenylether in Gegenwart von Bromwasserstoff und Wasserstoffperoxid umgesetzt wird. Bei dieser Umsetzung wird die Zielverbindung in besonderer Reinheit mit hoher Ausbeute erhalten.

## Beschreibung

Die Erfindung betrifft ein Verfahren zur Herstellung von Alkyl-4-bromphenylethern.

Es ist bekannt, Alkyl-4-bromphenylether durch Bromierung von Phenolethern herzustellen; beispielsweise Carter et al, J. Chem. Soc., 1948, 150, 153. Im allgemeinen wird hierbei Tetrachlormethan, Chloroform oder Eisessig als Lösungsmittel eingesetzt. Als Nebenprodukt entsteht bei dieser Umsetzung Bromwasserstoffsäure. Die beiden zuerst genannten Lösungsmittel sind cancerogen. Eisessig hat den Nachteil, daß er nicht recycliert werden kann, da es zur Aufarbeitung des Reaktionsgemisches mit Wasser verdünnt werden muß. Über die Isomerenreinheit der erzeugten Verbindungen ist wenig bekannt.

In der WO 92/13820 wird ein Verfahren zur Erzeugung von 4-Bromphenetol offenbart, bei dem eine wäßrige Phase aus der Umsetzung von Phenol mit Ethylbromid mit vorgereinigtem Ethylphenylether und Wasserstoffperoxid versetzt wird. Nach einer Behandlung der bei dieser Umsetzung gewonnenen organischen Phase mit Natriumbisulfitlösung erhält man mit einer Ausbeute von 77 % Ethyl-4-bromphenylether (oder 4-Bromphenetol) lediglich in 94,7 %-iger Reinheit. Aus dem in der WO 92/13820 beschriebenen Produktgemisch läßt sich durch übliche destillative Trennmethoden kein reineres 4-Bromphenetol gewinnen, weil hierbei stets das unerwünschte 2-Bromphenetol "mitgeschleppt" würde.

Das vorstehend beschriebene Verfahren hat den besonderen Nachteil, daß bei ihm große Mengen Natriumsulfat anfallen. Natriumsulfat kann nicht im Abwasser entsorgt werden, da es beispielsweise die Betonröhren in Kanalsystemen korrodiert; deshalb sind Abwassergrenzwerte für Sulfat allgemein üblich.

In neuerer Zeit wurden zwei weitere Verfahren zur Herstellung von Alkyl-4-bromphenylether publiziert, die zwar sehr hohe Ausbeuten liefern, jedoch wie nachfolgend erläutert, für eine technische Produktion ungeeignet sind.

Shoji Kajigaeshi et al (J. Chem. Soc. Per. Trans., 1990, 897) verwenden Benzyltrimethylammoniumtribromid als bromierendes Agens. Die Bromierung wird in sehr verdünnter Lösung in Methylenchlorid/Methanol durchgeführt. Die Aufarbeitung als Gemisch ist kompliziert. Das Bromierungsagens ist teuer und muß separat hergestellt werden, die anzuwendende Menge ist hoch. Für 0,5 g Anisol werden 1,98 g BTMABr₃ eingesetzt. Nach der Umsetzung verbleiben 1,17 g Benzyltrimethylammoniumbromid im Reaktionsgemisch. Dieser teure Stoff muß in irgendeiner nicht beschriebenen Weise zurückgewonnen werden.

Bravermann (US-A-4 517 388) verwendet Bromdimethylsulfoniumbromid als Bromierungsagens. Die erzielte Ausbeute von 102 % bei der Bromierung von Anisol deutet darauf hin, daß kein reines Produkt erhalten wurde.

Nachteilig bei diesem Verfahren ist vor allem die Bildung stöchiometrischer Mengen des äußerst geruchsbelästigenden Dimethylsulfids im Gemisch mit HBr und Methylenchlorid.

Der Erfindung lag nun die Aufgabe zugrunde, ein Verfahren zur Herstellung von Alkyl-4-Bromphenylethern bereitzustellen, das den folgenden Anforderungen genügt:
- Einfachheit,
- hohe Isomerenreinheit,
- hohe Ausbeute,
- Vermeidung von Abfallsalzen und organischen Lösungsmitteln.

Diese Probleme bzw. diese Aufgabe löst die Erfindung.

Erfindungsgemäß wird ein Verfahren zur Herstellung von Alkyl-4-Bromphenylethern bereitgestellt, bei dem der Alkylphenylether (korrespondierend zu dem Alkyl-4-Bromphenylether) unter Zugabe von Bromwasserstoff und Wasserstoffperoxid umgesetzt wird. Vorzugsweise wird dieses Verfahren so durchgeführt, daß dem Alkylphenylether oder einer Lösung des Alkylphenylethers das Wasserstoffperoxid und Bromwasserstoffsäure (wäßrige Lösung des Bromwasserstoffs) zugegeben werden. Es ist aber auch möglich, den Bromwasserstoff (HBr) zusammen mit dem Alkylphenylether vorzulegen und erst dann das Wasserstoffperoxid zuzugeben.

Die erfindungsgemäße Reaktion läuft nach der folgenden Reaktionsgleichung ab: 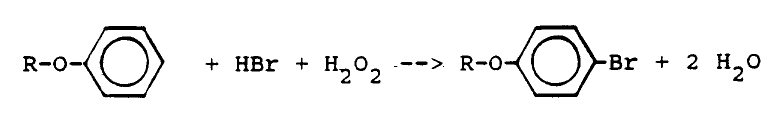
worin R in dem Alkylphenylether bzw. Alkyl-4-Bromphenylether für einen geradkettigen oder verzweigtkettigen Alkylrest mit 1 bis 4 Kohlenstoffatomen steht. Dieser Rest kann wiederum substituiert sein, beispielsweise durch einen Phenylrest oder Halogen. Vorzugsweise ist R Methyl oder Ethyl. Der aromatische Kern des Phenylethers kann durch einen weiteren Substituenten substituiert sein.

Eine Besonderheit des erfindungsgemäßen Verfahrens besteht darin, daß praktisch ohne jede weitere Zusätze gearbeitet werden kann (bspw. zusätzliche Säure) und daß die hohen Ausbeuten bei hoher Isomerenreinheit mittels einfacher destillativer Aufarbeitung des Reaktionsansatzes erhalten werden.

Im erfindungsgemäßen Verfahren kann der Alkylphenylether in Wasser, Bromwasserstoffsäure und/oder einem inerten organischen Lösungsmittel vorgelegt werden. Sodann wird unter Rühren Bromwasserstoffsäure und Wasserstoffperoxid zugegeben, wobei im Fall der Vorlage von Bromwasserstoffsäure und Alkylphenylether nur mit Wasserstoffperoxid versetzt werden muß.

Nach einer bevorzugten Ausführungsform des erfindungsgemäßen Verfahrens wird der reine Alkylphenylether vorgelegt und Bromwasserstoffsäure und Wasserstoffperoxid gleichzeitig zudosiert. Falls Bromwasserstoffgas zur Verfügung steht, kann dieses auch direkt ohne vorheriges Lösen in Wasser in den Ansatz eingeleitet werden, während H₂O₂ zudosiert wird.

Wasserstoffperoxid kann in allen handelsüblichen Konzentrationen, z.B. 30 %-ig, 50 %-ig, 70 %-ig, zum Einsatz kommen. Bromwasserstoffsäure kann ebenfalls in allen handelsüblichen Konzentrationen, z.B. 48 %-ig, 62 %-ig, eingesetzt werden.

Pro mol Alkylphenylether werden 1,02 bis 3 mol, vorzugsweise 1,03 bis 2 mol, insbesondere 1,05 bis 1,3 mol Bromwasserstoffsäure und 1,02 bis 1,6 mol, vorzugsweise 1,03 bis 1,4 mol, insbesondere 1,05 bis 1,3 mol Wasserstoffperoxid eingesetzt.

Als besonders günstig hat es sich erwiesen, HBr und H₂O₂ räumlich getrennt der Reaktionslösung zuzudosieren.

Die Umsetzung wird bei -10 bis +60°C, vorzugsweise -5 bis +30°C durchgeführt. Es ist aber auch möglich, zunächst bei niedrigerer Temperatur zu beginnen (beispielsweise -10 bis +20°C) und dann am Ende der Zugabe der weiteren Reaktionspartner die Temperatur ansteigen zu lassen (auf etwa 25 bis 30°C).

In manchen Fällen ist es günstig, den Alkylphenylether zunächst mit 1,0 bis 1,2 mol Bromwasserstoffsäure und H₂O₂ umzusetzen, dann die organische Phase abzutrennen und diese dann anschließend mit weiterem HBr/H₂O₂ zur Reaktion zu bringen.

Die Aufarbeitung erfolgt durch Phasentrennung und Destillation.

Bestehen die dem Reaktionskessel nachgeschalteten Anlagenteile aus Edelstahl, sollte das Reaktionsgemisch vor der Phasentrennung neutralisiert werden. Die Wasserphasen könnten zur Entfernung von Spuren Alkyl-4-Bromphenylether angestrippt und dann in einen Abwasserkanal abgelassen werden.

Der Alkyl-4-Bromphenylether wird schließlich durch destillative Aufarbeitung, vor allem der organischen Phase, gewonnen. Auf diese Weise erhält man die Zielverbindung in sehr hoher Reinheit mit sehr hohen Ausbeuten. Beispielsweise wurden mittels des erfindungsgemäßen Verfahrens 4-Bromphenetol und 4-Bromanisol in einer Reinheit von 99,9 % und Ausbeuten von über 90 % der Theorie gewonnen.

Die Vorteile des erfindungsgemäßen Verfahrens liegen insbesondere in seiner Einfachheit. Ausgehend von den eingesetzten Substanzen wird die Zielverbindung in wenigen Schritten erhalten. Die Verfahren des Standes der Technik erreichen beispielsweise nicht diese Kombination einer hohen Reinheit und hoher Ausbeute, ohne einen beträchtlichen Aufwand zur Abtrennung des Alkyl-4-Bromphenylethers. Ein weiterer Vorteil des erfindungsgemäßen Verfahrens besteht darin, daß dieses auch ohne organische Lösungsmittel betrieben werden kann. Vorteilhaft ist auch, daß das erfindungsgemäße Verfahren mit leicht zugänglichen Reaktanten durchgeführt wird. Bromwasserstoffsäure ist beispielsweise ein wohlfeiles Nebenprodukt beispielsweise aus der Produktion bromierter Flammschutzmittel. Schließlich ist auch das Wasserstoffperoxid als Oxidationsmittel einfach zugänglich und in dem erfindungsgemäßen Verfahren gut zu beherrschen. Wasserstoffperoxid wird in umweltschonenden Kreislaufverfahren (z.B. Antrachinonverfahren, Ullmann, 4. Auflage, Band 17, S. 697 - 709, Verlag Chemie) aus Wasserstoff und Luftsauerstoff hergestellt.

Die Zielverbindung Alkyl-4-Bromphenylether wird als Zwischenprodukt bei der Herstellung insbesondere insektizider Wirkstoffe benötigt. Bei dieser Herstellung ist es ganz besonders wichtig, daß der Alkyl-4-Bromphenylether in ausreichender Reinheit eingesetzt wird. Für das 4-Bromphenetol wird in der Literatur beispielsweise ein Schmelzpunkt von +4°C berichtet. Mit dem erfindungsgemäßen Verfahren ist es nun erstmalig gelungen, ein 4-Bromphenetol mit einem Schmelzpunkt von 11,5°C darzustellen. Dies bedeutet, daß erfindungsgemäß eine neue Verbindung bereitgestellt wird, zumindest soweit das 4-Bromphenetol angesprochen ist. Dieser Befund läßt sich damit erklären, daß im Stand der Technik eine vollständige Trennung der isomeren Produkte aus dem Herstellungsverfahren nicht möglich war. Somit ist es erfindungsgemäß erstmals möglich geworden das destillativ gewonnene Endprodukt Alkyl-4-Bromphenylether bei der Herstellung pharmazeutischer und insektizider Wirkstoffe einzusetzen, ohne daß zugleich eine unerwünschte Verunreinigung durch beispielsweise Alkyl-2-Bromphenylether zu befürchten ist.

Die Erfindung wird in den folgenden Beispielen näher erläutert.

### Beispiel 1

Zu 2.210 kg (18,09 kmol) Phenetol in einem Reaktionsgefäß wurden unter Rühren und Kühlen innerhalb von mindestens 6 h bei 3 bis 5°C 2.281 l (19,89 kmol) 48 %-ige Bromwasserstoffsäure und 1.127 l (19,89 kmol) 50 %-iges Wasserstoffperoxid gleichzeitig zudosiert. Am Ende der Dosierung wurde die Reaktionsmischung noch weitere 2 h bei 6 bis 8°C gerührt und dann auf 26°C aufgewärmt. Der Rührer wurde abgestellt und die untere organische Phase in ein anderes Gefäß überführt. Bei 24°C werden 29 l (0,25 kmol) 48 % HBr und 14,5 l (0,25 kmol) 50 %-iges H₂O₂ gleichzeitig zudosiert. Es wurde 6 h bei 26°C gerührt und dann wurde mit Natronlauge neutralisiert. Die untere organische Phase wurde abgetrennt und einmal mit 500 l Wasser gewaschen. Die vereinigten wäßrigen Phasen wurden dann angestrippt und in den Kanal gegeben. Eine geringe Menge im Destillat abgeschiedenes 4-Bromphenetol wurde mit der zuvor erhaltenen Hauptmenge vereinigt. Die Destillation im Vakuum liefert 3.381 kg (93 % der Theorie) an 99,9 %-igem 4-Bromphenetol mit einem Schmelzpunkt von 11,5°C.

### Beispiel 2

Zu 2.200 kg (18,0 kmol) Phenetol in einem Reaktionsgefäß wurden unter Rühren und Kühlen innerhalb von mindestens 6 h bei 18 - 20°C 2.377 l (20.7 kmol) 48 %-ige Bromwasserstoffsäure und 1.173 l (20,7 kmol) 50 %-iges H₂O₂ gleichzeitig zudosiert. Es wurde über 4 h auf über 25°C erwärmt und bei dieser Temperatur gleichzeitig 44 l (0,38 kmol) HBr und 22 l (0,38 kmol) H₂O₂ zudosiert. Der Ansatz wurde wie in Beispiel 1 beschrieben aufgearbeitet. Es wurden 3.292 kg (91 % der Theorie) an 99,9 %-igem 4-Bromphenetol erhalten.

### Beispiel 3

Zu 324,4 g (3 mol) Anisol wurden bei 4°C 371,5 ml (3,24 mol) 48 %-ige HBr und 193,5 ml (3,24 mol) 50 %-iges Wasserstoffperoxid innerhalb von 8,5 h zudosiert. Man ließ über Nacht nachreagieren und versetzte dann mit 11 ml 48 %-iger HBr und 5 ml Wasserstoffperoxid und rührte 6 h bei 26 - 28°C. Es wurde mit Natronlauge neutralisiert, die organische Phase wurde unten abgetrennt und diese mit Wasser gewaschen. Nach Destillation im Vakuum erhielt man 521 g (93 % der Theorie) an 99,9 %-igem 4-Bromanisol.

## Patentansprüche

1. Verfahren zur Herstellung von Alkyl-4-Bromphenylethern, dadurch gekennzeichnet, daß ein Alkylphenylether unter Zugabe von Bromwasserstoff und Wasserstoffperoxid umgesetzt wird.

2. Verfahren nach Anspruch 1, wobei die Umsetzung ohne Zusatz von Lösungsmitteln erfolgt.

3. Verfahren nach einem der vorstehenden Ansprüche, wobei der Alkylphenylether vorgelegt und durch Zugabe von Bromwasserstoffsäure und Wasserstoffperoxid zum Produkt umgesetzt wird.

4. Verfahren nach einem der vorstehenden Ansprüche, dadurch gekennzeichnet, daß pro mol Alkylphenylether mehr als 1 mol Wasserstoffperoxid und mehr als 1 mol HBr eingesetzt werden.

5. Verfahren nach einem der vorstehenden Ansprüche, wobei HBr und Wasserstoffperoxid gleichzeitig zu vorgelegtem Alkylphenylether zugegeben werden.

6. Verfahren nach einem der vorstehenden Ansprüche, wobei die Zudosierung der Reaktionspartner zum Alkylphenylether bei -10 bis +20°C erfolgt und im Anschluß daran auf 25 - 40°C erwärmt wird.

7. Verfahren nach einem der vorstehenden Ansprüche, wobei 1 mol Alkylphenylether zunächst mit 1,0 bis 1,2 mol Peroxid und HBr umgesetzt werden, dann die organische Phase abgetrennt und anschließend diese mit weiterem HBr/Wasserstoffperoxid zur Reaktion gebracht wird.
